# EUROPÄISCHE PATENTSCHRIFT

(11) **EP 3 820 413 B1**
(45) Veröffentlichungstag und Bekanntmachung des Hinweises auf die Patenterteilung: **24.12.2025**
(21) Anmeldenummer: 19739256.6
(22) Anmeldetag: 09.07.2019
(51) Int. Cl.: A61F 5/01

(54) **VORRICHTUNG ZUM DÄMPFEN EINER KÖRPERBEWEGUNG ÜBER EIN KÖRPERGELENK**
DEVICE FOR DAMPING A BODY MOVEMENT VIA A JOINT
DISPOSITIF D'AMORTISSEMENT D'UN MOUVEMENT CORPOREL PAR L'INTERMÉDIAIRE D'UNE ARTICULATION CORPORELLE

(30) Priorität: 09.07.2018 DE 102018116569
(43) Veröffentlichungstag der Anmeldung: 19.05.2021
(73) Patentinhaber: Betterguards Technology GmbH, 13585 Berlin (DE)
(72) Erfinder: BICHLER, Vinzenz, 10777 Berlin (DE); STUMPER, Timo, 10963 Berlin (DE); BUSCHINGER, Oscar, 10707 Berlin (DE)
(74) Vertreter: Okoampah, Rene
(86) Internationale Anmeldenummer: PCT/EP2019/068394
(87) Internationale Veröffentlichungsnummer: WO 2020/011780

(56) Entgegenhaltungen:
- EP-B1- 2 717 809
- WO-A1-2006/047906
- WO-A1-2006/047906
- DE-A1- 2 238 038
- US-A1- 2003 073 941
- US-A1- 2015 119 777
- US-A1- 2017 027 735

## Beschreibung

### Technisches Gebiet

Die vorliegende Erfindung betrifft eine Vorrichtung zum Dämpfen einer Körperbewegung über ein Körpergelenk.

### Stand der Technik

Es ist bekannt, die Körperbewegung über ein Körpergelenk mittels Vorrichtungen zu stabilisieren, um Traumata infolge eines Umknickens, das heißt einer Bewegung des Körpergelenks über zumindest eines seiner Körpergelenkachsen in einem unphysiologischen Bereich entgegenzuwirken. Die häufigste Form des Umknicktraumas ist eine Dehnung oder Ruptur eines Bandes oder Knochens infolge eines ansteigenden Gelenkbewegungswinkels, welcher in einer Änderung des Abstands zwischen erstem Körperteil und zweiten Körperteil resultiert, welche über das Körpergelenk verbunden sind, beispielsweise Hand und Unterarm. Bei Überschreiten eines bestimmten Gelenkwinkels oder einer bestimmten Gelenkwinkelgeschwindigkeit beziehungsweise Gelenkwinkelbeschleunigung kann es mithin zu Verletzungen des das Körpergelenk stabilisierenden Bandapparats oder zu Rupturen kommen.

Um dies zu verhindern, insbesondere um Traumata des Handgelenks infolge eines Sturzes, mithin einer Bewegung des Handgelenks über zumindest eines seiner Handgelenksachsen in einem unphysiologischen Bereich zu verhindern, sind Vorrichtungen bekannt, die in einem bestimmten Umfang Bewegungen ermöglichen und ab einem bestimmten Grenzwinkel der Bewegung um die Gelenkachse eine Bewegung gänzlich verhindern. Hierzu sind einerseits relativ starre Orthesen bekannt, bei denen das Hemmen von Bewegungen unter Verwendung von Schienen oder Schienenplatten im Vordergrund steht. Als Beispiel hierfür sind etwa Handgelenksorthesen oder Schienen für Handschuhe bekannt. Diese sollten die Bewegung des Handgelenks beim Sturz einschränken uns so ein Überstrecken des Körpergelenks verhindern. Die Schienen oder derartigen Orthesen für das Handgelenk weisen allerdings eine erhebliche Einschränkung der Bewegung auf, wodurch diese Systeme kaum genutzt werden und es daher zu zahlreichen Verletzungen in Form eines Überstreckens des Handgelenks kommt.

Ferner sind Vorrichtungen bekannt, bei welchen eine Bewegung des Körpergelenks bis zu einem bestimmten Grenzwinkel der Bewegung zugelassen wird, und aufgrund des Aufbaus ab diesem Grenzwinkel eine Bewegung gänzlich blockiert wird. Solch eine Vorrichtung ist beispielsweise aus der EP 2 717 809 B1 bekannt. Solche Vorrichtungen bieten vor Erreichen des Grenzwinkels keinerlei Schutzwirkung. Es ist jedoch bekannt, dass das Verletzungsrisiko beim Umknicken, insbesondere bei bereits geschwächten Bändern oder nach einer Verletzung, jedoch auch signifikant von der beim Umknicken auftretenden Winkelgeschwindigkeit und Winkelbeschleunigung und nicht allein vom Winkel abhängt. Im Bereich unterhalb des Grenzwinkels bieten solche Vorrichtungen keinerlei Schutz. Zudem ist nach Erreichen des Grenzwinkels die Bewegung komplett blockiert. Das abrupte Abstoppen der Umknickbewegung erzeugt eine große Belastung auf die Struktur des Körpergelenks, wodurch die Gefahr einer Verletzung, beispielsweise eine Ruptur am Knochen oder eine Knorpelverletzung erhöht wird. Durch dieses Blockieren des Körpergelenks wird zudem die Umknickbewegung auf das nächste Gelenk übertragen. Bei einem Umknicken über das Sprunggelenk ist dies das Kniegelenk, bei einem Umknicken über das Handgelenk entsprechend das Ellenbogengelenk oder das Schultergelenk. Aufgrund des vorliegenden großen Hebelarms, der ungünstigen Krafteinleitung und der Komplexität des Kniegelenks oder des Ellenbogengelenks beziehungsweise des Schultergelenks kann es dann zu schwerwiegenden Verletzungen, wie Kreuzbandrissen oder Meniskusschäden führen, welche sich aufgrund ihrer Einschränkung der Person, der Komplexität und der weniger guten Heilungsmöglichkeiten vielfach negativer Auswirken, als eine Sprunggelenksbänderverletzung beziehungsweise Handgelenksbänderverletzung.

Ferner sind Vorrichtungen bekannt, welche im angezogenen Zustand stets ein Mindestmaß an Bewegung zulassen, bei gefährlichen Bewegungen jedoch blockieren. Aus der DE 10 2014 107 335 A1 ist eine Vorrichtung bekannt, für welche mittels eines dilatanten Fluids eine geschwindigkeitsabhängige beziehungsweise beschleunigungsabhängige adaptive Rückhaltung bereitgestellt ist, wobei etwa eine Dorsalextension über das Handgelenk ein Ausziehen eines Auszugskörpers aus einer mit dem dilatanten Fluid gefüllten Aufnahme bewirkt. Aufgrund des geringen resultierenden Auszugsweges ist die wirksame Fläche des Auszugskörpers entsprechend groß auszuführen.

EP 0 564 734 A1 betrifft eine verstellbare Orthese.

DE 10 2012 011 433 A1 zeigt eine Vorrichtung mit dilatantem Material zur adaptiven Bewegungsbegrenzung.

US 2017/027735 A1 zeigt eine orthopädische Vorrichtung mit vorstehenden Elementen.

US 2015/119777 A1 zeigt eine Strebe und Zugfedern für eine Strebe.

US 2003/073941 A1 zeigt ein Verfahren und Vorrichtung zum Schutz des Epikondylus.

DE 22 38 038 A1 zeigt eine Vorrichtung zur Abstützung der beine eines Skifahrers bei extremer Rücklage.

WO 2006/047906 A1 zeigt einen Stützapparat für ein oder mehrere Körpergelenke.

### Darstellung der Erfindung

Ausgehend von dem bekannten Stand der Technik ist es eine Aufgabe der vorliegenden Erfindung, eine verbesserte Vorrichtung zum Dämpfen einer Körperbewegung über ein Körpergelenk bereitzustellen.

Die Aufgabe wird durch eine Vorrichtung zum Dämpfen einer Körperbewegung über ein Körpergelenk mit den Merkmalen des Anspruchs 1 gelöst. Vorteilhafte Weiterbildungen ergeben sich aus den Unteransprüchen, der Beschreibung und den beigefügten Figuren.

Entsprechend wird eine Vorrichtung zum Dämpfen einer Körperbewegung über ein Körpergelenk vorgeschlagen, umfassend einen ersten Teil zum Anbinden an einen ersten Körperteil, einen relativ zum ersten Teil schwenkbaren zweiten Teil zum Anbinden an einen in Bezug zum ersten Körperteil jenseits des Körpergelenks gelegenen zweiten Körperteil, ein an dem zweiten Teil befestigtes Dämpfungselement, und ein zugsteifes Verbindungselement, wobei sich das Verbindungselement von dem ersten Teil zum zweiten Teil erstreckt, und wobei das zugsteife Verbindungselement an dem ersten Teil angebunden ist und über das Dämpfungselement an dem zweiten Teil befestigt ist. Fernerhin ist ein Beabstandungselement zwischen dem ersten Teil und dem zweiten Teil angeordnet, wobei das Beabstandungselement derart ausgebildet ist, dass bei einer Schwenkbewegung des ersten Teils relativ zum zweiten Teil das Beabstandungselement das Verbindungselement von einem Drehpol der Schwenkbewegung beabstandet hält. Das Verbindungselement ist als Band ausgebildet, wobei das Band über das mindestens eine Beabstandungselement verläuft. Ferner verbindet eine biegsame Struktur zum Bereitstellen der Schwenkbarkeit des ersten Teils und des zweiten Teils relativ zueinander das erste Teil und das zweite Teil, wobei die biegsame Struktur in Form einer Platte ausgebildet ist.

Dadurch, dass ein Beabstandungselement zwischen dem ersten Teil und dem zweiten Teil angeordnet ist, wobei das Beabstandungselement derart ausgebildet ist, dass bei einer Schwenkbewegung des ersten Teils relativ zum zweiten Teil das Beabstandungselement das Verbindungselement in einen Abstand zu einem Drehpol der Schwenkbewegung hält, kann bei der Schwenkbewegung des ersten Teils relativ zum zweiten Teil in eine erste Schwenkrichtung eine Weglänge entlang des Verlaufs des Verbindungselements gesehen in Vergleich zu einer Weglänge, welche sich ohne das Beabstandungselement einstellen würde, vergrößert werden. Entsprechend ist der resultierende Auszugsweg, welchen das Dämpfungselement erfährt, vergrößert. Aufgrund des vergrößerten Auszugsweges ist es möglich, dass das Dämpfungselement eine effektive Dämpfung der Schwenkbewegung bereitstellen kann, und/oder kleiner ausgeführt werden kann als bei einer Ausführungsform ohne Beabstandungselement.

Als "Drehpol" ist vorliegend kein konstruktives Element zu verstehen, sondern derjenige Raumpunkt, um den der ersten Teil gegenwärtig relativ zum zweiten Teil dreht. Die Drehgeschwindigkeit im Drehpol ist im betrachteten Augenblick null. Mit anderen Worten ist unter "Drehpol" ein Drehpunkt beziehungsweise eine Drehachse zu verstehen, um welche(n) der ersten Teil relativ zum zweiten Teil dreht beziehungsweise schwenkt. Der Drehpol kann dabei stationär ausgebildet sein, mithin ein relativ zum ersten Teil und/oder zweiten Teil gleichbleibend angeordneter Drehpunkt beziehungsweise angeordnete Drehachse sein, oder aber eine sich seitlich ändernde Position relativ zum ersten Teil und/oder zum zweiten Teil aufweisen. Der Drehpol kann etwa als Momentanpol ausgebildet sein, und bevorzugt entsprechend eine Polbahn relativ zum ersten Teil und/oder zum zweiten Teil aufweisen.

Der Begriff "Köpergelenk" umfasst alle Gelenke eines Menschen oder Tieres. Ein Körpergelenk kann insbesondere ein Einzelgelenk eines Körpers sowie eine Anordnung mit mehreren Gelenkachsen sein, insbesondere das Handgelenk, das Sprunggelenk, das Kniegelenk, das Schultergelenk, das Hüftgelenk, das Ellenbogengelenk, mindestens ein Fingergelenk, oder mindestens ein Teilbereich der Wirbelsäule, wobei sowohl die gelenkige Verbindung zweier benachbarter Wirbel als auch ein größerer, mehrere Wirbel umfassender Bereich als "Köpergelenk" verstanden wird.

Bevorzugt ist das Dämpfungselement als geschwindigkeitsabhängig bzw. beschleunigungsabhängig wirkendes Dämpfungselement, ausgebildet, welches einen sprunghaften Anstieg der Widerstandskraft ermöglicht. In dieser Hinsicht wird Bezug genommen auf die Anmeldung EP 3238670 A1**.** Die Anmeldung EP 3 238 670 A1 zeigt ein geschwindigkeitsabhängiges Dämpfungselement.

Durch die mittels dem Dämpfungselement bereitgestellte geschwindigkeitsabhängige bzw. beschleunigungsabhängige Dämpfung ist der Grad der Dämpfung durch das Dämpfungselement abhängig von der Höhe der Geschwindigkeit, mit welcher das Verbindungselement an dem Dämpfungselement zieht, beziehungsweise von der Höhe der Beschleunigung, welche durch das Verbindungselement auf das Dämpfungselement wirkt. Der Sprunghafte anstieg der Widerstandskraft hat zur folge, dass bereits kleine Geschwindigkeitserhöhungen zu einer Erhöhung um ein Vielfaches der Widerstandskraft führen.

Das Dämpfungselement weist bevorzugt einen rohrförmigen ersten Dämpferteil auf, der fest mit dem zweiten Teil verbunden ist, und weist einen relativ zum ersten Dämpferteil entlang einer Auszugsrichtung, welche sich entlang der Längsachse des rohrförmigen ersten Dämpferteils erstreckt, beweglichen, zweiten Dämpferteil auf, der mit dem Verbindungselement, dem Band, verbunden ist. Der zweite Dämpferteil erstreckt sich teilweise im Inneren des rohrförmigen ersten Dämpferteils und weist darin einen Auszugskörper auf, mittels welchem im Wesentlichen die Dämpfungswirkung erzielt wird. In dem Dämpfungselement ist ferner ein Dämpfungsmedium enthalten. Alternativ kann auch der erste Dämpferteil mit dem Verbindungselement, dem Band, verbunden sein und der zweite Dämpferteil mit dem zweiten Teil.

Unter "rohrförmig" wird vorliegend eine hohle Struktur verstanden. Der Querschnitt kann dabei rund, oval oder eckig sein, ferner kann der Querschnitt in eine erste Erstreckungsrichtung größer sein als in einer zweite Erstreckungsrichtung.

Das Dämpfungselement kann gemäß einer nicht beanspruchten Ausführungsform eine Dämpfungskraft bereitstellen, welche abhängig ist von einer Dämpfungskonstante des Dämpfungselements und der Geschwindigkeit im Dämpfungselement. Die Dämpfungskraft ist dabei unabhängig vom Weg, beispielsweise der aktuellen Elongation des Dämpfungselements.

Wenn gemäß einer nicht beanspruchten Weiterbildung das Dämpfungselement adaptiv wirkend ist, kann es zudem bis zu einer vorgegebenen Grenz-Geschwindigkeit und/oder Grenz-Beschleunigung eine erste, geringe Dämpfungskonstante aufweisen, und ab der vorgegebenen Grenz-Geschwindigkeit und/oder Grenz-Beschleunigung eine höhere Dämpfungskonstante aufweisen.

In einer nicht beanspruchten Weiterbildung ist es möglich, dass mehrere Grenz-Geschwindigkeiten und/oder Grenz-Beschleunigungen vorgegeben sind und entsprechend die Höhe der

Dämpfungskonstante sich mehrfach erhöht. Dadurch ist es möglich, dass bei Körperbewegungen um das zu dämpfenden Körpergelenk, welche Geschwindigkeiten und/oder Beschleunigungen unterhalb der vorgegebenen Grenzwerte im Dämpfungselement erzeugen, die Dämpfungswirkung des Dämpfungselements gering ist und somit die Beeinflussung und Dämpfung dieser Körperbewegungen gering ist. Erfolgt eine Körperbewegung im oder in der Nähe eines unphysiologischen Bereichs, kann das adaptive Dämpfungselement derart ausgebildet, sein, dass die darin erzeugten Geschwindigkeiten und/oder Beschleunigungen zumindest einen der Grenzwerte übersteigen und das Dämpfungselement folglich eine hohe Dämpfungswirkung bereitstellt, so dass die Körperbewegung stark gedämpft und dadurch ein Verletzungsrisiko infolge dieser Bewegung vermindert oder gar gänzlich verhindert werden kann. Durch diese adaptive Dämpfungswirkung ist es möglich, im physiologischen Bereich Körperbewegungen lediglich gering oder nahezu überhaupt nicht zu beeinträchtigen, und im und/oder nahe des unphysiologischen Bereichs Körperbewegungen stark zu dämpfen, um so das Verletzungsrisiko der die Vorrichtung tragenden Person zu minimieren.

In einer weiteren nicht beanspruchten Ausführungsform kann das Dämpfungselement eine Feder zum Bereitstellen einer Federwirkung, besonders bevorzugt eine Zugfeder und/oder Druckfeder, aufweisen. Hierdurch kann es ermöglicht werden, die Dämpfungswirkung ferner von einem Auszugsweg abhängig auszubinden.

Dadurch dass das Verbindungselement als zugsteifes Band ausgebildet ist, wobei das Band über das mindestens eine Beabstandungselement verläuft, ist es möglich, die Vorrichtung sehr genau an die zugrundeliegende Anatomie an und um das Körpergelenk herum anzupassen. Das Band zeichnet sich dadurch aus, dass es einerseits zugsteif ist, mithin Zugkräfte übertragen kann, und andererseits quer zur Längserstreckung des Bandes biegeweich beziehungsweise flexibel ist, so dass es quer zu seiner Längserstreckung an eine entsprechende Struktur anlegbar ist. Zudem kann ein Band in einfacher Weise umgelenkt werden. Der Begriff Band umfasst vorliegend allgemein ein längliches, biegeschlaffes, elastisches Element, welches die Form einer einzelnen Faser, eines Faserstrangs, eines Drahts, einer Kordel, eines Seils, eines Textilgewebes mit begrenzter Breite und festen, beidseitigen Webkanten oder dergleichen aufweisen kann.

Alternativ kann das Verbindungselement auch als zugsteifes Stabelement ausgebildet sein. Bevorzugt ist das Stabelement dabei einstückig mit einem zweiten Dämpferteil des Dämpfungselements ausgebildet.

Gemäß einer nicht beanspruchten Ausführungsform ist ein Gelenk zum Bereitstellen der Schwenkbarkeit des ersten Teils und des zweiten Teils relativ zueinander zwischen dem ersten Teil und dem zweiten Teil angeordnet, bevorzugt ein zumindest Querkraft und/oder Normalkraft übertragendes Gelenk, besonders bevorzugt ein Drehgelenk. Dadurch ist es möglich, dem Drehpol relativ zum ersten Teil und zum zweiten Teil eine feste räumliche Position zuzuweisen. Mit anderen Worten liegt der Drehpol in der Drehachse des Gelenks. Dies erlaubt eine genaue Vorbestimmung der durch eine Gelenksbewegung hervorgerufenen Weglänge des Verbindungselementes, des Bandes, und mithin einer Auszugslänge am Dämpfungselement.

Bevorzugt weist die Vorrichtung eine Gelenkanordnung auf, welche mindestens zwei Gelenke umfasst. Dabei ist ein erstes Gelenk der Gelenkanordnung derart ausgebildet, dass es eine Bewegung in eine zu dämpfende Bewegungsrichtung zulässt, und ein zweites Gelenk beabstandet zu dem ersten Gelenk angeordnet, wobei das zweite Gelenk derart ausgebildet ist, dass es eine Bewegung entgegen der zu dämpfenden Bewegungsrichtung zulässt und bevorzugt derart, dass es ab einem vorgegebenen Grenzwinkel einer Bewegung in die zu dämpfende Bewegungsrichtung blockiert ist. Dadurch kann einerseits eine freie Beweglichkeit entgegen der zu dämpfenden Bewegungsrichtung, andererseits eine effektive Dämpfungswirkung in Richtung der zu dämpfenden Bewegung bereitgestellt werden.

Unter "Bewegungsrichtung" wird vorliegend sowohl eine lineare Bewegungsrichtung als auch ein Drehbewegungsrichtung, sowie eine Bewegungsrichtung, ausgebildet aus einer Mischung aus einer geradlinigen und einer gekrümmte Bewegungsbahn verstanden.

Die Dämpfung der zu dämpfenden Bewegungsrichtung ist dabei über das zugsteife Verbindungselement, das Band, in Verbindung mit dem Dämpfungselement und dem Beabstandungselement bereitgestellt. Das Beabstandungselement ist derart angeordnet, dass es das Verbindungselement, das Band, in einem Abstand zum ersten Gelenk hält und umlenkt. Bei einer Schwenkbewegung in Richtung der zu dämpfenden Bewegungsrichtung erfährt das Verbindungselement, das Band, dabei eine Verschiebung über das Beabstandungselement, wobei die Größe der Verschiebung abhängig ist zu einem Abstand zwischen dem ersten Gelenk und einem Anbindungspunkt und/oder einem Umlenkpunkt, an welchem das Band an dem beziehungsweise über das Beabstandungselement umgelenkt wird.

Dadurch, dass eine biegsame Struktur zum Bereitstellen der Schwenkbarkeit des ersten Teils und des zweiten Teils relativ zueinander bereitgestellt ist, welche das erste Teil und das zweite Teil verbindet, kann die Vorrichtung einen besonders einfachen Aufbau aufweisen.

Bevorzugt sind die biegsame Struktur, das erste Teil und/oder das zweite Teil einstückig ausgebildet.

Alternativ oder zusätzlich kann die biegsame Struktur gemäß einer weiteren nicht beanspruchten Ausführungsform auch aus einer Mehrzahl von aneinander gereihten Einzelelementen ausgebildet sein, wobei das Verbindungselement, das Band, bevorzugt durch jedes der Einzelelemente geführt ist, wobei die Einzelelemente bevorzugt durch das Verbindungselement, das Band, zusammengehalten sind, wobei bevorzugt jeweils zwei Einzelelemente miteinander gelenkig verbunden sind. Eine derart ausgebildete Vorrichtung kann besonders gut an die Anatomie der Körperregion, in welchem sich das zu dämpfende Körpergelenk befindet, angepasst werden.

Gemäß einer weiteren bevorzugten Ausführungsform ist die Anbindung des Verbindungselements, des Bandes, am ersten Teil in Form einer Befestigung des Verbindungselements, des Bandes, an dem ersten Teil ausgebildet. Hierdurch kann ein besonders einfacher Aufbau erzielt werden.

Alternativ kann die Anbindung des Verbindungselements, des Bandes, am ersten Teil in Form einer Rückführung vom ersten Teil zurück zum zweiten Teil ausgebildet sein, wobei die Rückführung derart ausgebildet ist, dass das Verbindungselement, das Band, am ersten Teil umgelenkt und zum zweiten Teil rückgeführt und daran befestigt ist. Bevorzugt weist die Rückführung ein reibungsarmes Material auf. Auch kann die Rückführung in Form eines Flaschenzuges bereitgestellt sein, wodurch sich die bei einer Bewegung des ersten Teils relativ zum zweiten Teil einstellende Längenänderung des Verbindungselements, des Bandes, nochmals vergrößern kann.

Um eine besonders steife Struktur sowie einen einfachen Aufbau Vorrichtung bereitzustellen, kann ein Beabstandungselement, über welchen das Band verläuft, gemäß einer weiter bevorzugten Ausführungsform an dem ersten Teil angeordnet sein und bevorzugt einteilig mit dem ersten Teil ausgebildet sein, und/oder kann ein Beabstandungselement, über welchen das Band verläuft, an dem zweiten Teil angeordnet sein und bevorzugt einteilig mit dem zweiten Teil ausgebildet sein.

Gemäß einer weiter bevorzugten Ausführungsform ist ein Beabstandungselement separat zu dem ersten Teil und dem zweiten Teil bereitgestellt und bevorzugt zwischen dem ersten Teil und dem zweiten Teil angeordnet. Dies erlaubt eine nochmals genauere Anpassung der Vorrichtung hinsichtlich der anatomischen Rahmenbedingungen, welche sich über die Körperregionen im Bereich des Körpergelenks ergeben.

Wenn ein Beabstandungselement gemäß einer weiteren bevorzugten Ausführungsform als Vorsprung ausgebildet ist, der sich bevorzugt von dem ersten Teil, bevorzugt über das Körpergelenk und/oder ein den ersten Teil und den zweiten Teil schwenkbar verbindendes Gelenk und/oder eine den ersten Teil und den zweiten Teil schwenkbar verbindende biegsame Struktur, zum zweiten Teil erstreckt oder der sich von dem zweiten Teil, bevorzugt über das Körpergelenk und/oder ein den ersten Teil und den zweiten Teil schwenkbar verbindendes Gelenk und/oder eine den ersten Teil und den zweiten Teil schwenkbar verbindende biegsame Struktur, zum ersten Teil erstreckt, kann zum einen in einfacher Weise eine sichere Beabstandung des Bandes und/oder einer vom Drehpol beabstandeten Umlenkung des Bandes erzielt werden. Zudem kann der Vorsprung derart angeordnet sein, dass er ferner als Schiene oder Protektorelement fungiert, um beispielsweise bei einem Sturz und einem daraus resultierenden Aufprall zumindest ein Teil der resultierenden Aufprallkraft durch den Vorsprung zu absorbieren.

Eine besonders vorteilhafte Ausgestaltung der Vorrichtung ergibt sich, wenn diese als Vorrichtung zum Dämpfen einer Bewegung über das Handgelenk, bevorzugt zum Dämpfen einer Dorsalextension über das Handgelenk, ausgebildet ist.

Bevorzugt ist der ersten Teil zum Anbinden an die Hand distal des Handgelenks ausgebildet und der zweiten Teil zum Anbinden an den Unterarm proximal des Handgelenks ausgebildet, oder der zweiten Teil ist zum Anbinden an die Hand distal des Handgelenks ausgebildet und der erste Teil zum Anbinden an den Unterarm proximal des Handgelenks ausgebildet.

Alternativ kann die Vorrichtung zum Dämpfen einer Körperbewegung über das Sprunggelenk, das Knie, die Schulter, den Ellenbogen, oder einem weiteren Gelenk eines Körpers, bevorzugt eines menschlichen Körpers, ausgebildet sein.

Die zu dämpfende Bewegungsrichtung kann ein Schwenken über das Körpergelenk, sowie eine Rotation, beispielsweise entlang der Wirbelsäule, umfassen. Bevorzugt ist die Vorrichtung derart ausgebildet, dass sie eine Flexion der Hand erlaubt und eine Extension blockiert. Alternativ kann die Vorrichtung der ausgebildet sein, dass sie eine Extension der Hand erlaubt und eine Flexion blockiert.

Gemäß einer weiteren nicht beanspruchten Ausführungsform kann die Vorrichtung einen Einstellmechanismus zum Einstellen einer Länge des Verbindungselementes, des Bandes, aufweisen. Dadurch kann die Vorrichtung sehr genau an die Anatomie des zu schützenden Körpergelenks und der daran anschließenden Körperteile und/oder den jeweiligen Anwendungsfall angepasst werden.

Wenn gemäß einer weiteren nicht beanspruchten Ausführungsform das zugsteife Verbindungselement, das Band, über eine Führung geführt ist, kann die Position des zugsteifen Verbindungselement, des Bandes, entlang seiner Erstreckung besonders genau vorgegeben werden.

Gemäß einer weiteren nicht beanspruchten Ausführungsform ist eine Rückstellung, bevorzugt in Form eines elastischen Bandes oder einer Feder vorgesehen, welche eine Rückstellung des ersten Teils relativ zum zweiten Teil in eine vorgegebene Ausgangslage ermöglicht.

Als reibungsarme Materialien, beispielsweise für die Führung oder Kontaktpunkte zwischen Band und Beanstandungselement, können bevorzugt ein schmierfähiges Polymer, ein Metall oder ein Polymer beziehungsweise eine Polymerschicht aus PTFE oder POM oder einem anderen geeigneten Kunststoff Anwendung finden.

Gemäß einer weiteren nicht beanspruchten Ausführungsform kann sich das Band in mehrere Abschnitte fächerförmig aufteilen, sodass der Anbindungsbereich des Bandes entsprechend vergrößert ist.

Ein besonders vorteilhafter Aufbau der Vorrichtung lässt sich erzielen, wenn eine Mehrzahl von Beabstandungselementen vorgesehen ist.

Gemäß einer weiteren nicht beanspruchten Ausführungsform weist die Führung mindestens einen Kanal, bevorzugt eine Vielzahl von Kanälen zum Durchführen des Bandes auf. Die Kanäle weisen bevorzugt eine gekrümmte Erstreckung und/oder eine Innenfläche mit einem geringen Reibungskoeffizienten auf. An den Enden eines Kanals kann dieser einen glockenförmigen Austritt aufweisen, um das Ein- und Ausgleiten des Bandes zu erleichtern und zu verhindern, dass das Band an einer Kante fest klemmt.

Gemäß einer bevorzugten Weiterbildung umfasst der erste Teil und/oder der zweite Teil mindestens eine Auflagefläche zum flächigen Kontaktieren des ersten beziehungsweise des zweiten Körperteils. Durch eine flächige Auflage an einem Körperteil kann einer Weichteilverformung entgegengewirkt werden. Die Auflagefläche ist derart gestaltet, dass die Vorrichtung nicht in die Weichteilstruktur eines Körperteils wie zum Beispiel eines Gelenks oder Muskels, eingedrückt werden, d.h. eintauchen kann. Ein Eintauchen der Vorrichtung in die Weichteilstruktur eines Körperteils hätte zur Folge, dass bei einer Körperbewegung dem Dämpfungselement eine vergleichsweise geringere Weglänge zur Verfügung steht.

Gemäß einer weiter bevorzugten Ausführungsform ist die Vorrichtung eine Handgelenkorthese, wobei der erste Teil zur Befestigung an einer Hand eines Anwenders ausgebildet ist und der zweite Teil zur Befestigung an einem Unterarm des Anwenders ausgebildet ist. Der erste Teil ist dazu an Form und Abmessungen einer menschlichen Hand angepasst. Der zweite Teil ist dazu an Form und Abmessungen eines menschlichen Unterarms angepasst. Auf diese Weise lassen sich äußerliche Kräfte, die abrupt auf das Handgelenk einwirken, im Rahmen der vorstehend Beschriebenen Eigenschaften dämpfen.

### Kurze Beschreibung der Figuren

Bevorzugte weitere Ausführungsformen der Erfindung werden durch die nachfolgende Beschreibung der Figuren näher erläutert. Dabei zeigen:
Figur 1 schematisch eine Seitenansicht einer Vorrichtung zum Dämpfen einer Körperbewegung über ein Körpergelenk gemäß einer ersten nicht anspruchsgemäßen Ausführungsform;
Figur 2 schematisch eine Seitenansicht einer Vorrichtung zum Dämpfen einer Körperbewegung über ein Körpergelenk gemäß einer zweiten nicht anspruchsgemäßen Ausführungsform;
Figur 3 schematisch eine Seitenansicht einer Vorrichtung zum Dämpfen einer Körperbewegung über ein Körpergelenk gemäß einer dritten nicht anspruchsgemäßen Ausführungsform;
Figur 4 schematisch eine weitere Seitenansicht der Vorrichtung aus Figur 3;
Figur 5 schematisch eine weitere Seitenansicht der Vorrichtung aus Figur 3;
Figur 6 schematisch eine Seitenansicht einer Vorrichtung zum Dämpfen einer Körperbewegung über ein Körpergelenk gemäß einer vierten Ausführungsform;
Figur 7 schematisch eine Seitenansicht einer Vorrichtung zum Dämpfen einer Körperbewegung über ein Körpergelenk gemäß einer fünften Ausführungsform;
Figur 8 schematisch eine Seitenansicht einer Vorrichtung zum Dämpfen einer Körperbewegung über ein Körpergelenk gemäß einer sechsten Ausführungsform;
Figur 9 schematisch eine Seitenansicht einer biegsamen Struktur einer Vorrichtung zum Dämpfen einer Körperbewegung über ein Körpergelenk gemäß einer weiteren nicht beanspruchten Ausführungsform;
Figur 10 schematisch eine weitere Seitenansicht der Vorrichtung aus Figur 9;
Figur **11** schematisch eine weitere Seitenansicht der Vorrichtung aus Figur 9;
Figur 12 schematisch eine Seitenansicht einer biegsamen Struktur einer Vorrichtung zum Dämpfen einer Körperbewegung über ein Körpergelenk gemäß einer weiteren nicht beanspruchten Ausführungsform;
Figur 13 schematisch eine Seitenansicht einer biegsamen Struktur einer Vorrichtung zum Dämpfen einer Körperbewegung über ein Körpergelenk gemäß einer weiteren nicht beanspruchten Ausführungsform;
Figur 14 schematisch eine weitere Seitenansicht der Vorrichtung aus Figur 13; und
Figur 15 schematisch eine Seitenansicht einer Vorrichtung zum Dämpfen einer Körperbewegung über ein Körpergelenk gemäß einer weiteren nicht beanspruchten Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen. Ferner wird auf eine wiederholte Beschreibung dieser Elemente teilweise verzichtet, um Redundanzen zu vermeiden.

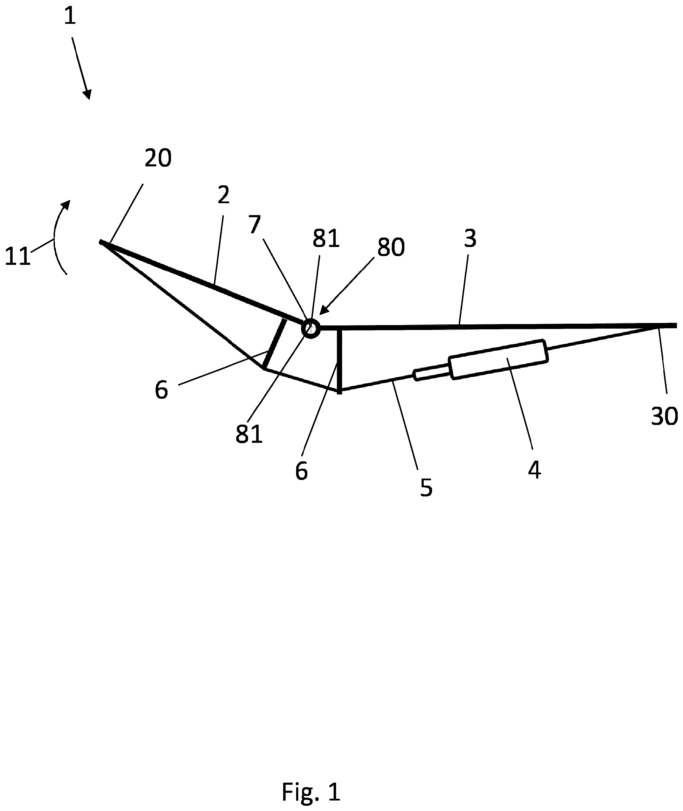

In Figur 1 ist schematisch eine Vorrichtung 1 zum Dämpfen einer Körperbewegung über ein Körpergelenk gemäß einer ersten nicht beanspruchten Ausführungsform gezeigt. Die Vorrichtung 1 umfasst einen ersten Teil 2 zur Anbindung an einen ersten Körperteil und einen relativ zum ersten Teil 2 schwenkbaren zweiten Teil 3 zum Anbinden an einen in Bezug zum ersten Körperteil jenseits des Körpergelenks gelegenen zweiten Körperteil. An dem zweiten Teil 3 ist ein Dämpfungselement 4 an einer Anbindung 30 befestigt. Zwischen dem ersten Teil 2 und dem zweiten Teil 3 erstreckt sich ein Verbindungselement in Form eines zugsteifen Bandes 5. Das Band 5 ist an einem der Anbindung 30 gegenüber gelegenen Ende an einer Anbindung 20 an dem ersten Teil 2 befestigt.

Der ersten Teil 2 und der zweiten Teil 3 sind über ein Gelenk 80 in Form eines Drehgelenks verbunden, worüber eine Schwenkbarkeit des ersten Teils 2 relativ zum zweiten Teil 3 bereitgestellt ist. Die Vorrichtung 1 weist ferner zwei Beabstandungselemente 6 auf, über welche das Band 5 verläuft, wobei die Beabstandungselemente 6 derart ausgebildet sind, dass bei einer Schwenkbewegung des ersten Teils 2 relativ zum zweiten Teil 3 die Beabstandungselemente 6 das Band 5 in einem Abstand zu einem Drehpol 7 der Schwenkbewegung halten, wie weiter unten näher erläutert.

Der Drehpol 7 zwischen dem ersten Teil 2 und dem zweiten Teil 3 ist vorliegend ein stationärer Drehpol 7, welcher durch die Gelenkachse 81 des Gelenks 80 bereitgestellt ist.

Das Dämpfungselement 4 weist einen rohrförmigen ersten Dämpferteil 40 auf, der fest mit dem zweiten Teil 3 verbunden ist, und weist einen relativ zum ersten Dämpferteil 40 entlang einer Auszugsrichtung, welche sich entlang der Längsachse des rohrförmigen ersten Dämpferteils 40 erstreckt, beweglichen, zweiten Dämpferteil 42 auf, der mit dem Band 5 verbunden ist. Der zweite Dämpferteil 42 erstreckt sich teilweise im Inneren des rohrförmigen ersten Dämpferteils 40 und weist darin einen Auszugskörper (nicht gezeigt) auf. In dem Dämpfungselement 4 ist ferner ein Dämpfungsmedium enthalten. Alternativ kann auch der erste Dämpferteil 40 mit dem Band 5 verbunden sein und der zweite Dämpferteil 42 mit dem zweiten Teil 3.

Das Dämpfungselement 4 ist ferner adaptiv wirkend ausgebildet. Mithin stellt es bis zu einer vorgegebenen Grenzgeschwindigkeit und einer vorgegebenen Grenzbeschleunigung des Auszugskörpers relativ zum ersten Dämpferteil 40 eine geringe Dämpfungswirkung bereit und stellt jenseits der Grenzwerte eine höhere Dämpfungswirkung bereit. Die vorgegebenen Grenzwerte können beispielsweise einem Dilatanzsprung eines im ersten Dämpferteil 40 aufgenommenen dilatanten Fluids entsprechen, oder konstruktiv am Auszugskörper, beispielsweise über das Vorsehen eines Federelements an einem Durchlasskanal, vorgegeben werden.

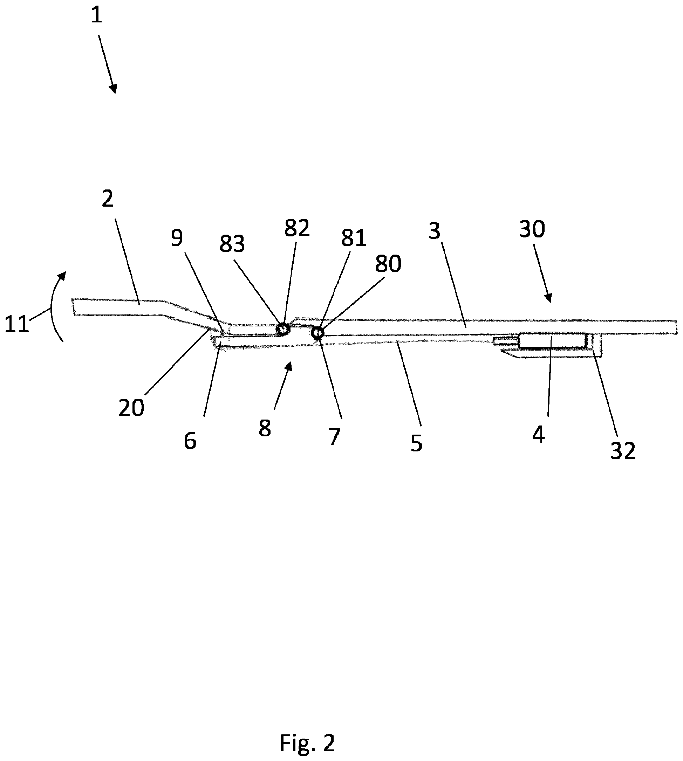

Figur 2 zeigt schematisch eine Seitenansicht einer Vorrichtung 1 zum Dämpfen einer Körperbewegung über ein Körpergelenk gemäß einer zweiten nicht beanspruchten Ausführungsform in einer Ausgangslage. Die Vorrichtung 1 entspricht im Wesentlichen der in Figur 1 gezeigten Vorrichtung, wobei statt eines Gelenks 80 vorliegend eine Gelenkanordnung 8, umfassend ein erstes Gelenk 80 und ein davon beabstandetes zweites Gelenk 82, vorgesehen ist. Der erste Teil 2 ist über das erste Gelenk 80 in Richtung einer zu dämpfenden Bewegungsrichtung **11** schwenkbar mit dem zweiten Teil 3 verbunden. Mithin stellt die Drehachse 81 des ersten Gelenks 80 den stationären Drehpol 7 der Schwenkbewegung bereit. Ferner ist ein vorderer Abschnitt des ersten Teils 2 gegenüber einem in Figur 2 durch den zweiten Teil 3 verdeckten hinteren Abschnitt des ersten Teils 2 entgegen der Bewegungsrichtung **11** schwenkbar. Ein Schwenken des ersten Teils 2 relativ zum zweiten Teil 3 aus der gezeigten Ausgangslage heraus in Richtung der Bewegungsrichtung **11** erfolgt mithin über das erste Gelenk 80.

An dem zweiten Teil 3 ist ein Dämpfungselement 4 befestigt, von welchem sich entsprechend Figur 1 ein zugsteifes Band 5 zum ersten Teil 2 erstreckt, an welchem es an der Anwendung 20 fixiert ist.

Der zweiten Teil 3 weist ferner ein als Vorsprung ausgebildetes Beabstandungselement 6 auf, über welches das Band 5 verläuft. Das Beabstandungselement 6 ist derart ausgebildet, dass bei einer Schwenkbewegung des ersten Teils 2 relativ zum zweiten Teil 3 in Richtung der Bewegungsrichtung **11** das Band 5 in einem Abstand zu dem Drehpol 7 gehalten ist. Mit anderen Worten erfolgt eine Umlenkung des Bandes 5 über die Spitze des Vorsprungs. Das Beabstandungselement 6 ist einstückig mit dem zweiten Teil 3 ausgebildet.

Das als Vorsprung ausgebildet Beabstandungselement 6 erstreckt sich von dem zweiten Teil 3 über das erstes Gelenk 80 zum ersten Teil 2, sodass in der Ausgangslage, wie in Figur 2 gezeigt, eine freie Weglänge des Bandes 5 zwischen dem Ende des Vorsprungs und der Anbindung 20 gering ist.

Das Dämpfungselement 4 ist in einer Aufnahme 32 des zweiten Teils 3 aufgenommen, welche das Dämpfungselement 4 von äußeren Einflüssen, insbesondere von Stößen oder Schlägen schützt.

Die Vorrichtung 1 ist vorliegend zum Dämpfen einer Körperbewegung über das Handgelenk eines Menschen bzw. Anwenders ausgebildet. Hierfür weist die Vorrichtung 1 eine nicht gezeigte Anbindung am ersten Teil 2 zum Anbinden an eine Hand sowie eine nicht gezeigte Anbindung am zweiten Teil 3 zum Anbinden an einen Unterarm auf.

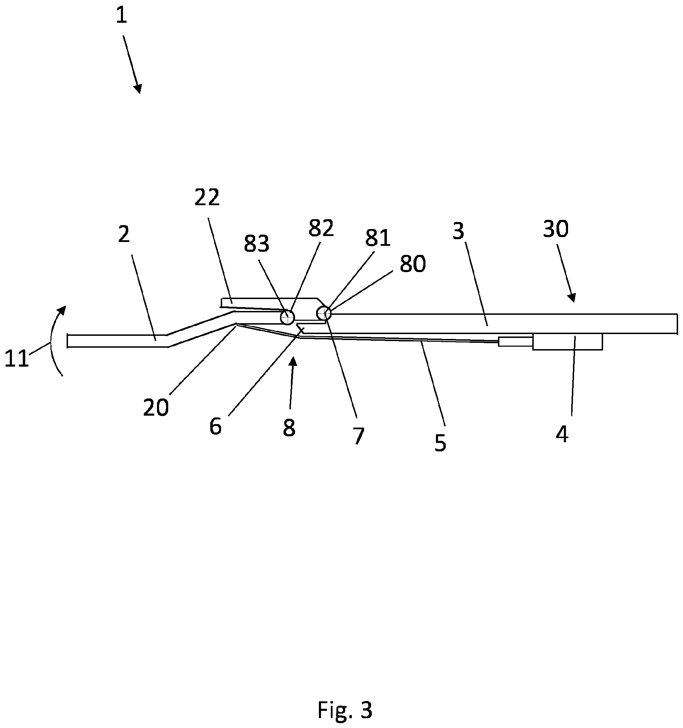

Figur 3 zeigt schematisch eine Seitenansicht einer Vorrichtung 1 zum Dämpfen einer Körperbewegung über ein Körpergelenk gemäß einer dritten nicht beanspruchten Ausführungsform. Die Vorrichtung 1 entspricht im Wesentlichen den vorgenannten Vorrichtungen, wobei bei der in Figur 3 gezeigten Vorrichtung 1 zwischen dem ersten Teil 2 und dem zweiten Teil 3 ein Zwischenelement 22 vorgesehen ist.

Das Zwischenelement 22 und der zweiten Teil 3 sind über das erste Gelenk 80 schwenkbar miteinander verbunden. Fernerhin sind das Zwischenelement 22 und der erste Teil 2 über das zweite Gelenk 82 schwenkbar miteinander verbunden. Das Zwischenelement 22 weist einen Anschlag 23 auf, welcher ein Schwenken des zweiten Teils relativ zum Zwischenelement 22 über die in Figur 3 gezeigte Ausgangslage hinaus in Richtung der zu dämpfenden Bewegungsrichtung 11 verhindert. Ein Schwenken entgegen der Bewegungsrichtung 11 aus der Ausgangslage wird dadurch nicht behindert.

Dadurch, dass der Anschlag 23 ein Schwenken des zweiten Teils 3 relativ zum Zwischenelement 22 über das zweite Gelenk 82 in Richtung der Bewegungsrichtung 11 verhindert, erfolgt in Bewegungsrichtung 11 aus der Ausgangslage heraus ein Schwenken des ersten Teils 2 und des Zwischenelements 22 gemeinsam über das erstes Gelenk 80 relativ zum weiten Teil 3. Mithin ist in Bewegungsrichtung 11 gesehen die Achse 81 des erstes Gelenks 80 der stationäre Drehpol 7 zwischen dem ersten Teil 2 und dem zweiten Teil 3.

Der zweiten Teil 3 weist ebenfalls ein Beabstandungselement 6 auf, über welchen das Band 5 verläuft. Das Beabstandungselement 6 ist wiederum ein sich einstückig von dem zweiten Teil 3 über das Gelenk 80 zum beziehungsweise in Richtung des ersten Teils 2 erstreckender Vorsprung, wobei das Beabstandungselement 6 zudem als Anschlag für das Zwischenelement 22 fungiert, sodass ein Schwenken des Zwischenelements 22 über das Gelenk 80 entgegen der Bewegungsrichtung 11 über die in Figur 3 gezeigte Ausgangslage hinaus verhindert ist.

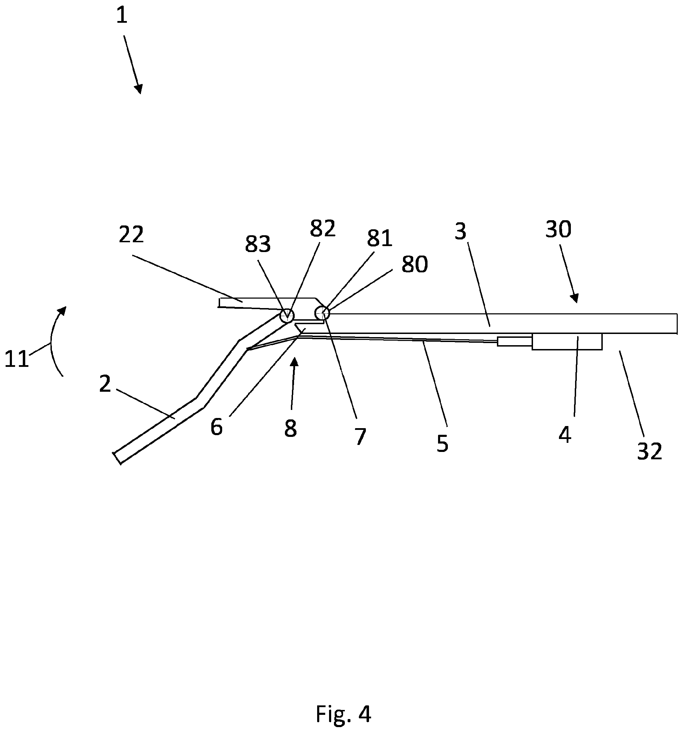

In Figur 4 ist schematisch eine weitere Seitenansicht der Vorrichtung aus Figur 3 gezeigt, in welcher der erste Teil 2 bezogen auf die in Figur 3 gezeigte Ausgangslage über das zweite Gelenk 82 relativ zum zweiten Teil 3 entgegen der Bewegungsrichtung 11 geschwenkt ist. Wie oben erläutert, erfolgte das Schwenken des ersten Teils 2 über die zweite Gelenkachse 83, da das Beabstandungselement 6 als Anschlag, beziehungsweise als Bewegungsbegrenzung, für das Zwischenelement 22 wirkt. Ein derart geartetes Schwenken des ersten Teils 2 und des zweiten Teils 3 relativ zueinander hat keine Auswirkungen auf das Dämpfungselement 4. Die Schwenkbewegung ist mithin nicht gedämpft.

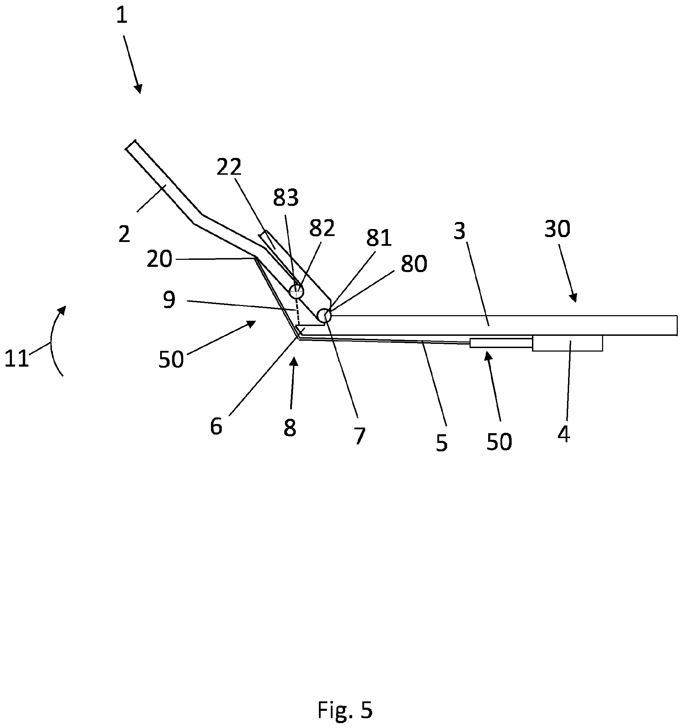

Figur 5 zeigt schematisch eine weitere Seitenansicht der Vorrichtung 1 aus Figur 3 in einer bezogen auf die in Figur 3 gezeigte Ausgangslage in Richtung der Bewegungsrichtung 11 geschwenkten Stellung. Mit anderen Worten ist der ersten Teil 2 relativ zum zweiten Teil 3 über das erste Gelenk 80 und somit den Drehpol 7 in Richtung der Bewegungsrichtung 11 geschwenkt.

Dadurch, dass das Beabstandungselement 6 das Band 5 beabstandet von dem Drehpol 7 hält beziehungsweise umlenkt, hat das Band 5 im Vergleich zu der in Figur 3 gezeigten Stellung eine Verschiebung relativ zum zweiten Teil 3 weg vom Dämpfungselement 4 erfahren, da sich eine Weglänge 50 zwischen der Anbindung 20 und der Spitze des Beabstandungselements 6 verlängert hat. Entsprechend der Verlängerung der Weglänge 50 erfolgt eine Dehnung beziehungsweise ein Ausziehen des Dämpfungselements 4, sodass die vorgeschriebene Bewegung in Bewegungsrichtung 11 durch das Dämpfungselement 4 gedämpft wird.

Zwischen dem zweiten Teil 3 und dem ersten Teil 2 ist ferner eine Rückstellung 9 infolge eines elastischen Bandes bereitgestellt, welches den ersten Teil 2 in Richtung der in Figur 3 gezeigten Ausgangslage zurückstellt, sobald eine in Bewegungsrichtung 11 wirkende Kraft kleiner ist als die durch die Rückstellung 9 auf das ersten Teil 2 wirkende Kraft.

Die in den Figuren 3 bis 5 gezeigte Vorrichtung 1 ist zum Dämpfen einer Dorsalextension über das Handgelenk eines Menschen ausgebildet. Hierfür weist der erste Teil 2 eine hier nicht gezeigte Anbindung an eine Hand und der zweite Teil 3 eine hier nicht gezeigte Anbindung an einen Unterarm auf.

Alternativ kann die in den Figuren 3 bis 5 gezeigte Vorrichtung 1 eine Schiene einer herkömmlichen Handgelenks-Orthese oder eines herkömmlichen Handgelenk-Schutzhandschuhs ersetzen.

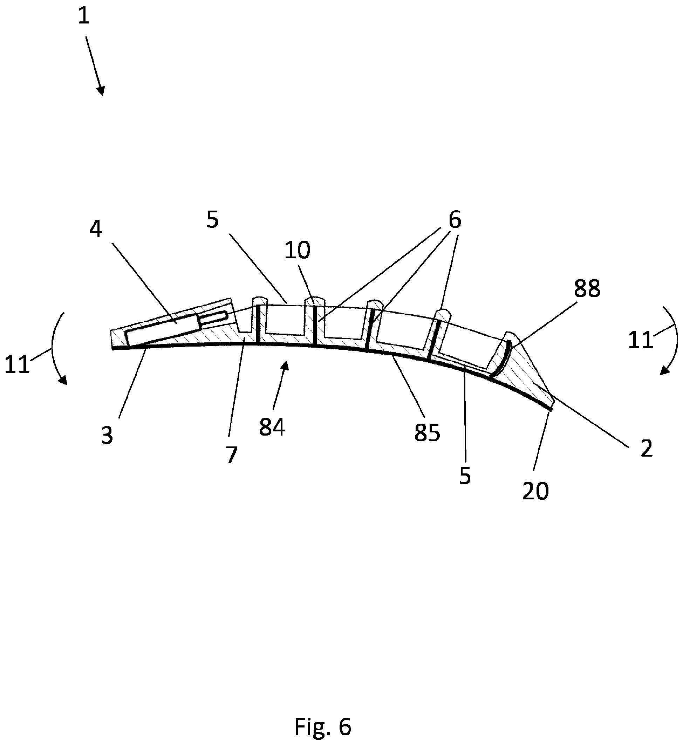

Figur 6 zeigt schematisch eine Seitenansicht einer Vorrichtung 1 zum Dämpfen einer Körperbewegung über ein Körpergelenk gemäß einer vierten Ausführungsform.

Die Vorrichtung 1 weist einen ersten Teil 2 zum Anbinden an einen ersten Körperteil, einen relativ zum ersten Teil 2 schwenkbaren zweiten Teil 3 zum Anbinden an einen in Bezug zum ersten Körperteil jenseits des Gelenks gelegenen zweiten Körperteil, ein an dem zweiten Teil 3 befestigtes Dämpfungselement 4, und ein zugsteifes Band 5 auf, wobei sich das Band 5 von dem ersten Teil 2 zum zweiten Teil 3 erstreckt, und wobei das zugsteife Band 5 mit einem Ende an dem ersten Teil 2 befestigt ist und an einem zweiten Ende über das Dämpfungselement 4 an dem zweiten Teil 3 befestigt ist.

Um eine Schwenkbarkeit des ersten Teils 2 relativ zum zweiten Teil 3 bereitzustellen, ist eine biegsame Struktur 84 vorgesehen, welche das erste Teil 2 und das zweite Teil 3 miteinander verbindet.

Die biegsame Struktur 84, das erste Teil 2 und das zweite Teil 3 sind einstückig ausgebildet, wobei die biegsame Struktur 84 in Form einer Platte 85 ausgebildet ist. Die Platte 85 kann beispielsweise ein Metall oder einen Kunststoff, bevorzugt einen faserverstärkten Kunststoff wie kohlenstofffaserverstärkter Kunststoff oder glasfaserverstärkter Kunststoff, aufweisen. Dadurch, dass die Dicke der Platte 85 relativ zur Länge zwischen dem ersten Teil 2 und dem zweiten Teil 3 dünnwandig ist, weist die Platte 85 entlang in der Länge gesehen eine entsprechend der Materialeigenschaften ausgebildete Elastizität auf. Dadurch ist ein Biegen in der Bewegungsrichtung 11 wie in Figur 6 gezeigt möglich.

Zwischen dem ersten Teil 2 und dem zweiten Teil 3 sind auf der Platte 85 eine Reihe von Beanstandungselementen 6 angeordnet, welche sich im Wesentlichen orthogonal von der Platte 85 weg erstrecken und über welche das zugsteife Band 5 von dem an dem zweiten Teil 3 befestigten Dämpfungselement 4 kommend über eine Umlenkung 88 an dem zweiten Teil angebunden ist, und über eine Führung 10 nahe der Platte 85 zum zweiten Teil 3 zurückgeführt und daran befestigt ist.

Ein Schwenken des zweiten Teils 3 relativ zum ersten Teil 2 erfolgt durch ein Verbiegen der Platte 85 in Richtung der Bewegungsrichtung 11, wobei sich der Drehpol im Sinne eines sich zeitlich verändernden Momentanpols ausbildet, welcher entsprechend der Krümmung der Platte 85 entlang einer Polbahn (nicht gezeigt) mit zunehmender Biegung wandert. Die Beabstandungselemente 6 halten das Band 5 in Bezug auf einen Verlauf des Bandes 5 ohne Beabstandungselemente 6 derart, dass ein vergrößerter Abstand zwischen dem Drehpol und dem Band 5 bereitgestellt ist.

Entsprechend ist bei einem Biegen der Vorrichtung 1 in Bewegungsrichtung 11 eine Weglänge des Bandes 5 vergrößert. Entsprechend erfährt das Dämpfungselement 4 eine Längenänderung beziehungsweise einen Zug in Richtung des ersten Teils 2, sodass die Bewegung in Bewegungsrichtung 11 durch das Dämpfungselement 4 gedämpft wird.

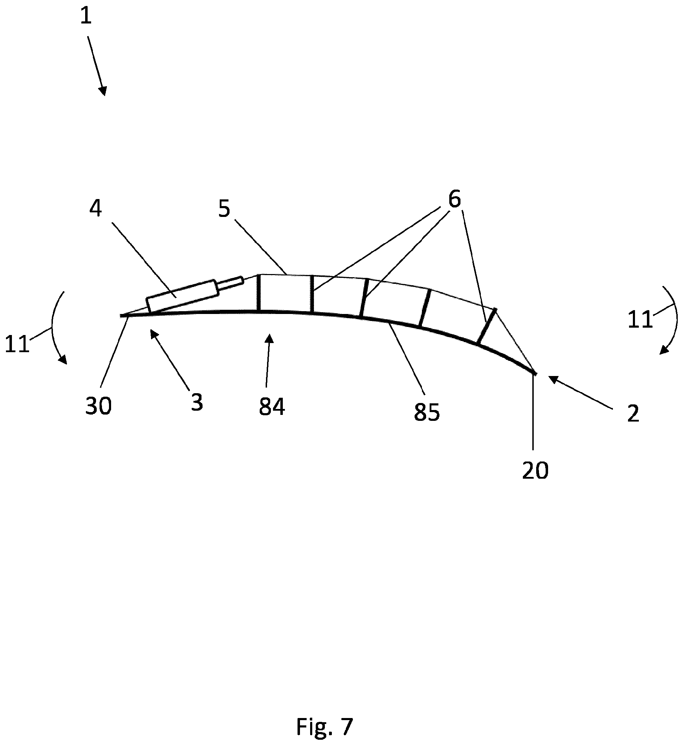

Figur 7 zeigt schematisch eine Seitenansicht einer Vorrichtung 1 zum Dämpfen einer Körperbewegung über ein Körpergelenk gemäß einer fünften Ausführungsform. Diese entspricht im Wesentlichen der Vorrichtung 1 aus Figur 6, wobei das zugsteife Band 5 an dem ersten Teil 2 über die Anbindung 20 befestigt ist. Fernerhin weist die Vorrichtung 1 eine Vielzahl von Beabstandungselementen 6 auf. Die Dämpfung der Bewegung in Bewegungsrichtung 11 erfolgt analog wie zu Figur 6 beschrieben.

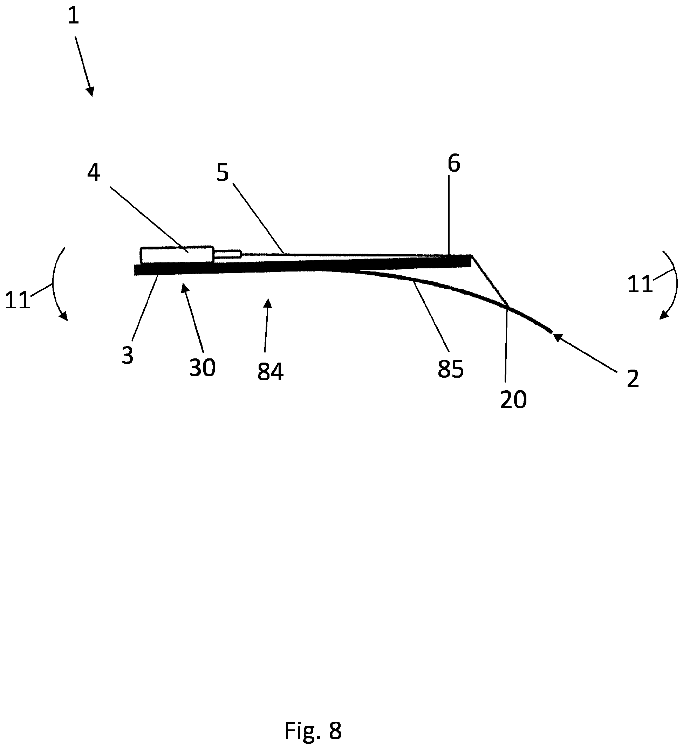

Figur 8 zeigt schematisch eine Seitenansicht einer Vorrichtung 1 zum Dämpfen einer Körperbewegung über ein Körpergelenk gemäß einer weiteren Ausführungsform. Diese entspricht im Wesentlichen der in Figur 7 gezeigten Ausführungsform, wobei statt der Vielzahl an sich orthogonal von der Platte 85 erstreckenden Beanstandungselementen 6 ein Beabstandungselement 6 in Form eines einstückig mit dem zweiten Teil 3 ausgebildeten, sich in Richtung des zweiten Teil erstreckenden Vorsprungs vorgesehen ist.

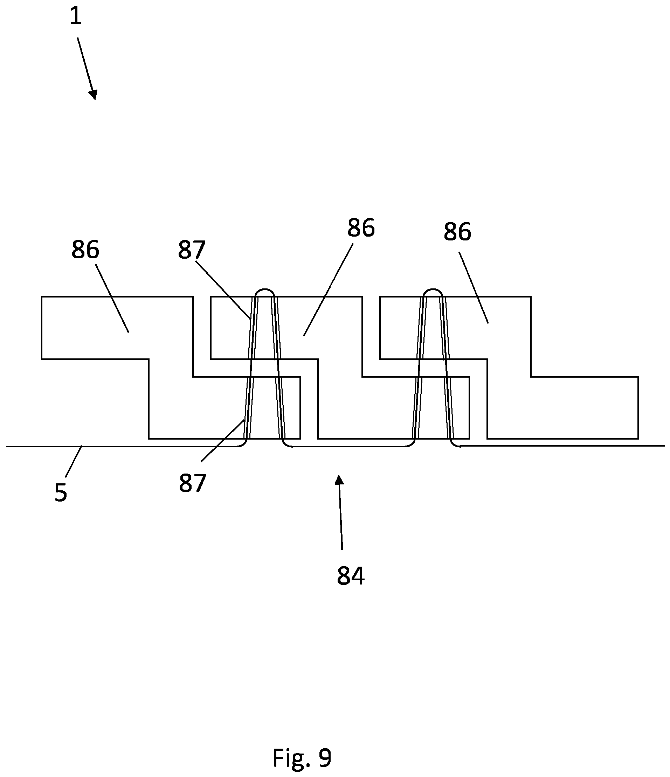

Figur 9 zeigt schematisch eine Seitenansicht einer biegsamen Struktur 84 einer Vorrichtung 1 zum Dämpfen einer Körperbewegung über ein Körpergelenk gemäß einer weiteren nicht beanspruchte Ausführungsform. Die biegsame Struktur 84 ist aus einer Mehrzahl von aneinander gereihten Einzelelementen 86 ausgebildet, wobei das Band 5 durch jedes der Einzelelemente 86 in hierfür vorgesehenen Durchgängen 87 geführt ist, wobei die Einzelelemente 86 durch das Band 5 zusammengehalten werden.

Um die Reibung des Bandes 5 gegenüber den Einzelelementen 86 gering zu halten, sind die Durchgänge 87 mit einem reibungsverringernden Überzug versehen. Alternativ können die Einzelelemente 86 ein Material mit geringem Reibungskoeffizienten aufweisen.

Die hier gezeigte biegsame Struktur 84 kann beispielsweise die biegsame Struktur 84 der Vorrichtung 1 aus Figur 7 ersetzen.

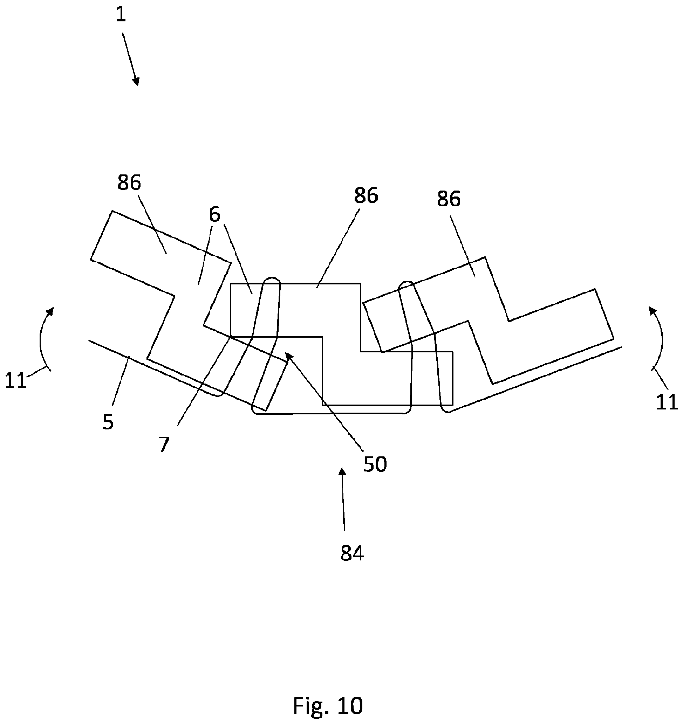

In Figur 10 ist schematisch eine weitere Seitenansicht der Vorrichtung 1 aus Figur 9 gezeigt. Die Einzelelemente 86 haben gegenüber der in Figur 9 gezeigten Lage der Vorrichtung 1 ein Schwenken in Richtung der Bewegungsrichtung 11 erfahren. Dabei können zwei benachbarte Einzelelemente 86 relativ zueinander zu einem sich an einem Kontaktpunkt zwischen diesen Einzelelementen 86 einstellenden Drehpol 7 schwenken. Durch das Schwenken über den Drehpol 7 erfolgt eine Verlängerung der Weglänge des Bandes 5 mit größer werdendem Winkel zwischen den beiden benachbarten Einzelelementen 86. Diese Verlängerung der Weglänge des Bandes 5 wird an das an dem zweiten Teil 3 angeordnete Dämpfungselement 4 übertragen, sodass die Bewegung in Bewegungsrichtung 11 durch das Dämpfungselement 4 gedämpft werden kann.

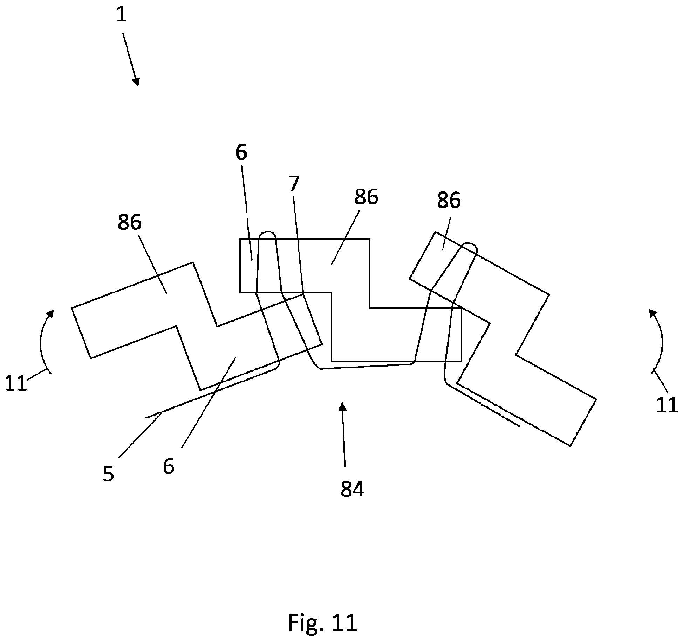

Figur 11 zeigt schematisch eine weitere Seitenansicht der Vorrichtung aus Figur 9. durch die biegsame Struktur 84 gemäß dieser Ausführungsform kann auch eine Bewegung entgegengesetzt der hinsichtlich Figur 10 beschriebenen Bewegung gedämpft werden. Auch in der in Figur 11 gezeigten Bewegungsrichtung 11 erfolgt durch ein Schwenken zweier benachbarter Einzelelemente 86 relativ zueinander über den sich einstellenden Drehpol 7 eine Verlängerung der Weglänge des Bandes 5, sodass das Dämpfungselement 4 ebenso eine Verlängerung erfährt, mithin eine Dämpfungswirkung entfalten kann, welche der Bewegung in Bewegungsrichtung 11 entgegenwirkt.

Die Einzelelemente 86 beziehungsweise deren Durchgänge 87 fungieren mithin als Beabstandungselemente 6.

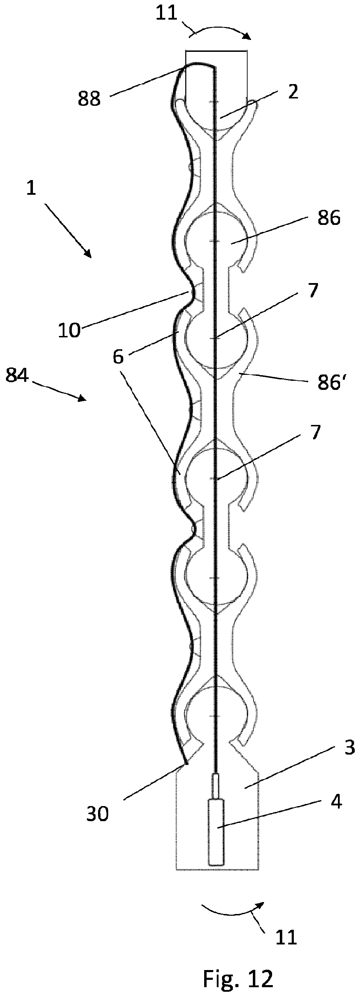

Figur 12 zeigt schematisch eine Seitenansicht einer biegsamen Struktur 84 einer Vorrichtung 1 zum Dämpfen einer Körperbewegung über ein Körpergelenk gemäß einer weiteren nicht beanspruchte Ausführungsform. Die biegsame Struktur 84 ist aus einer Mehrzahl von aneinander gereihten, komplementär zueinander ausgebildeten Einzelelementen 86, 86' ausgebildet, wobei das Band 5 an jedem der Einzelelemente 86, 86' geführt ist, wobei jeweils zwei Einzelelemente 86, 86' miteinander kugelgelenkig verbunden sind.

Das zugsteife Band 5 ist vorliegend an dem zweiten Teil 3 befestigt, läuft außenseitig über die biegsame Struktur 84 bis zum ersten Teil 2, wo es an einer Umlenkung 88 zum zweiten Teil 3 zurückgeführt wird. Bei einer Bewegung in Bewegungsrichtung 11, was beispielsweise einer Krümmung eines Rückens des Menschen entspricht, erfolgt eine Verlängerung des Weges des in der Führung 10 geführten Bandes 5 zwischen der Anbindung 30 und dem Dämpfungselement 4.

Die als Gelenkpfannen ausgebildeten Einzelelemente 86 wirken dabei als Beabstandungselemente 6 zu dem pro Gelenkverbindung vorliegenden Drehpol 7.

Die Führung 10 ist als eine Vielzahl von an jedem der Einzelelemente 68, 68' ausgebildeten Kanäle ausgebildet. Die Kanäle weisen eine gekrümmte Erstreckung auf. Die Innenfläche der Kanäle weist einen geringen Reibungskoeffizienten auf. An den Enden eines jeden Kanals kann dieser einen glockenförmigen Austritt aufweisen, um das Ein- und Ausgleiten des Bandes zu erleichtern und zu verhindern, dass das Band an einer Kante fest klemmt.

Demgemäß stellen die Einzelelemente 86, 86' Beabstandungselemente 6 dar, welche separat zu dem ersten Teil 2 und dem zweiten Teil 3 bereitgestellt sind und zwischen dem ersten Teil 2 und dem zweiten Teil 3 angeordnet sind.

Die Einzelelemente 86, 86' können in ihrer Längserstreckung den Wirbeln einer Wirbelsäule des Menschen nachempfunden sein, so dass die in Figur 12 gezeigte Vorrichtung 1 beispielsweise zum Dämpfen einer Bewegung über die Wirbelsäule eingesetzt werden kann.

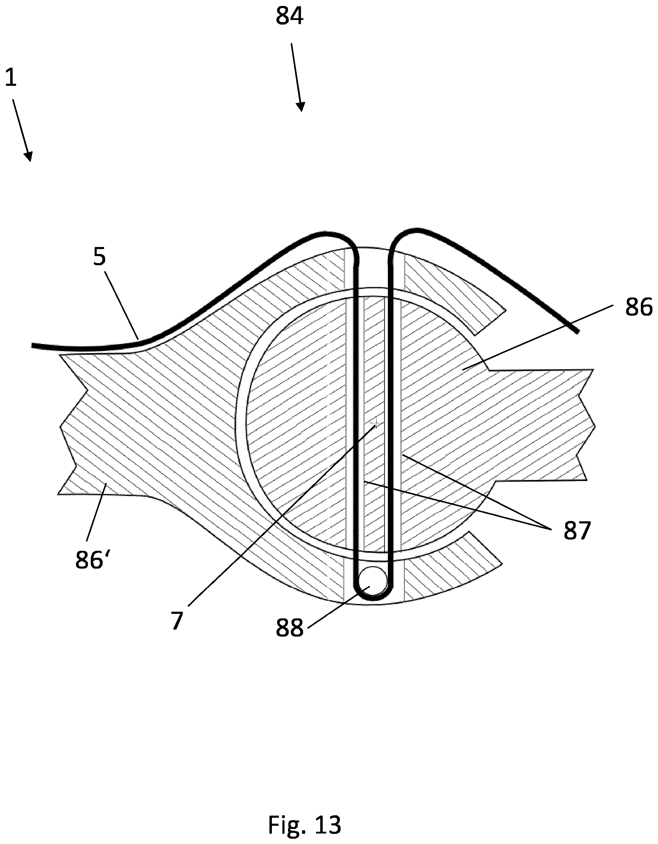

Figur 13 zeigt schematisch eine Seitenansicht einer biegsamen Struktur 84 einer Vorrichtung 1 zum Dämpfen einer Körperbewegung über ein Körpergelenk gemäß einer weiteren nicht beanspruchte Ausführungsform. Die biegsame Struktur 84 entspricht im Wesentlichen der in Figur 12 gezeigten Strukur. Das zugsteife Band 5 ist hierbei jedoch bei jeder Gelenkverbindung durch die Gelenkkugel der Einzelelemente 86' geführt.

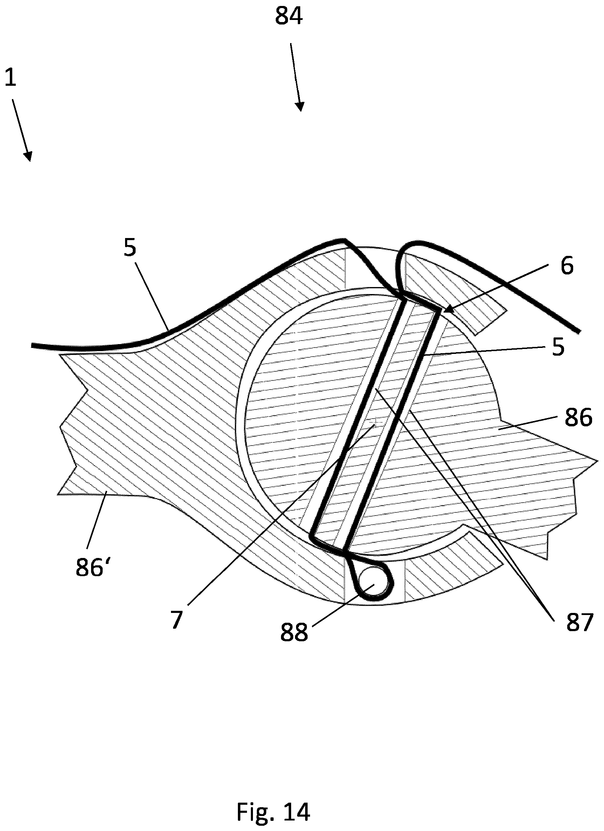

Erfolgt ein Schwenken zweier benachbarter Einzelelemente 86,68' zueinander, wie in Figur 14 angedeutet, so wirken die Enden der Durchgänge 87 der Gelenkkugeln der Einzelelemente 68' als Beabstandungselemente 6, sodass das Band eine Orientierung wie mit dem Bezugszeichen 5' angedeutet, und mithin eine Verlängerung der Weglänge, erfährt. Diese Verlängerung der Weglänge wird wiederum auf das Dämpfungselement 4 übertragen, sodass dieses eine Dämpfungswirkung auf die Schwenkbewegung ausüben kann.

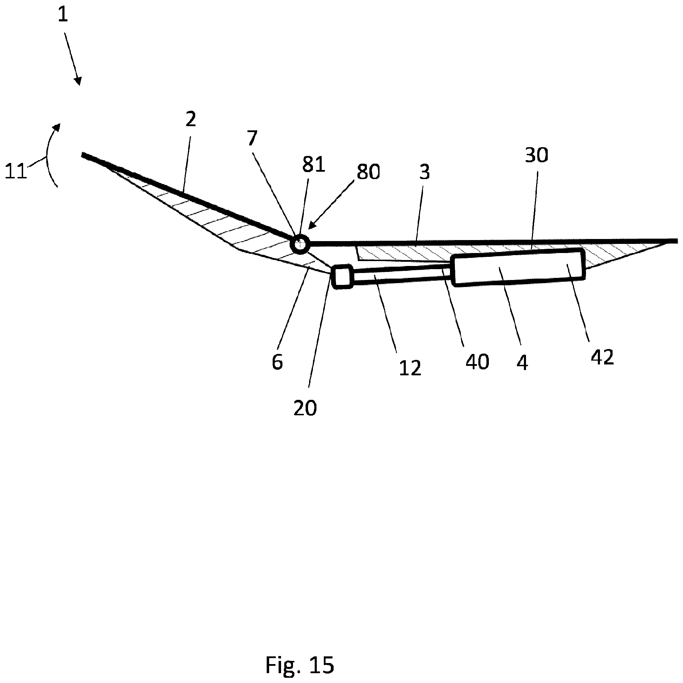

Figur 15 zeigt schematisch eine Seitenansicht einer Vorrichtung 1 zum Dämpfen einer Körperbewegung über ein Körpergelenk gemäß einer weiteren nicht beanspruchte Ausführungsform. Die Vorrichtung 1 umfasst einen ersten Teil 2 zum Anbinden an einen ersten Körperteil, einen relativ zum ersten Teil 2 schwenkbaren zweiten Teil 3 zum Anbinden an einen in Bezug zum ersten Körperteil jenseits des Körpergelenks gelegenen zweiten Körperteil, ein an dem zweiten Teil 3 befestigtes Dämpfungselement 4, und ein zugsteifes Verbindungselement in Form eines Stabelements 12, wobei sich das Stabelement 12 von dem ersten Teil 2 zum zweiten Teil 3 erstreckt, und wobei das zugsteife Verbindungselement an dem ersten Teil 2 angebunden ist und über das Dämpfungselement 4 an dem zweiten Teil (3) befestigt ist. Ein Beabstandungselement 6 ist zwischen dem ersten Teil 2 und dem zweiten Teil 3 angeordnet, wobei das Beabstandungselement 6 einstückig mit dem ersten Teil 2 ausgebildet ist und ferner derart ausgebildet ist, dass bei einer Schwenkbewegung des ersten Teils 2 relativ zum zweiten Teil 3 das Beabstandungselement 6 das Verbindungselement von einem Drehpol 7 der Schwenkbewegung beabstandet hält.

Der Drehpol 7 ist vorliegend relativ zum ersten Teil 2 und zweiten Teil 3 gesehen als stationärer Drehpol 7 bereitgestellt, welcher durch die Drehachse 81 eines Gelenks 80 definiert ist, welches zum Bereitstellen der Schwenkbarkeit des ersten Teils 2 und des zweiten Teils 3 relativ zueinander zwischen dem ersten Teil 2 und dem zweiten Teil 3 angeordnet ist.

Das Dämpfungselement 4 weist einen rohrförmigen ersten Dämpferteil 40 auf, der fest mit dem zweiten Teil 3 verbunden ist, und weist einen relativ zum ersten Dämpferteil 40 entlang einer Auszugsrichtung, welche sich entlang der Längsachse des rohrförmigen ersten Dämpferteils 40 erstreckt, beweglichen, zweiten Dämpferteil 42 auf, der mit dem Stabelement 12 verbunden ist. Der zweite Dämpferteil 42 erstreckt sich teilweise im Inneren des rohrförmigen ersten Dämpferteils 40 und weist darin einen Auszugskörper (nicht gezeigt) auf. In dem Dämpfungselement 4 ist ferner ein Dämpfungsmedium enthalten.

Das Dämpfungselement 4 ist ferner adaptiv wirkend ausgebildet. Mithin stellt es bis zu einer vorgegebenen Grenzgeschwindigkeit und einer vorgegebenen Grenzbeschleunigung des Auszugskörpers relativ zum ersten Dämpferteil 40 eine geringe Dämpfungswirkung bereit und stellt jenseits der Grenzwerte eine höhere Dämpfungswirkung bereit. Die vorgegebenen Grenzwerte können beispielsweise einem Dilatanzsprung eines im ersten Dämpferteil 40 aufgenommenen dilatanten Fluids entsprechen, oder konstruktiv am Auszugskörper einstellbar sein.

Das Stabelement 12 ist vorliegend einstückig mit dem zweiten Dämpferteil 42 des Dämpfungselements 4 ausgebildet. Alternativ kann das Stabelement auch an dem ersten Dämpferteil 40 des Dämpfungselements 4 angeordnet werden, wenn der zweiten Dämpferteil 42 des Dämpfungselements 4 mit dem zweiten Teil 3 verbunden ist.

Das ein Beabstandungselement 6 ist ferner als Vorsprung ausgebildet, der sich der sich von dem zweiten Teil 3 über das Gelenk 80 zum ersten Teil 2 erstreckt.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 1: Vorrichtung
- 2: Erster Teil
- 20: Anbindung
- 22: Zwischenelement
- 23: Anschlag
- 3: Zweiter Teil
- 30: Anbindung
- 32: Aufnahme
- 4: Dämpfungselement
- 40: Erster Dämpferteil
- 42: Zweiter Dämpferteil
- 5, 5': Band
- 50: Weglänge
- 6: Beanstandungselement
- 7: Drehpol
- 8: Gelenkanordnung
- 80: Gelenk
- 81: Gelenkachse
- 82: Zweites Gelenk
- 83: Zweite Gelenkachse
- 84: Biegsame Struktur
- 85: Platte
- 86, 86': Einzelelement
- 87: Durchgang
- 88: Umlenkung
- 9: Rückstellung
- 10: Führung
- 11: Bewegungsrichtung
- 12: Stabelement

## Patentansprüche

1. Vorrichtung (1) zum Dämpfen einer Körperbewegung über ein Körpergelenk, umfassend einen ersten Teil (2) zum Anbinden an einen ersten Körperteil, einen relativ zum ersten Teil (2) schwenkbaren zweiten Teil (3) zum Anbinden an einen in Bezug zum ersten Körperteil jenseits des Körpergelenks gelegenen zweiten Körperteil, ein an dem zweiten Teil (3) befestigtes Dämpfungselement (4), und ein zugsteifes Verbindungselement, wobei sich das Verbindungselement von dem ersten Teil (2) zum zweiten Teil (3) erstreckt, und wobei das zugsteife Verbindungselement an dem ersten Teil (2) angebunden ist und über das Dämpfungselement (4) an dem zweiten Teil (3) befestigt ist, wobei mindestens ein Beabstandungselement (6) zwischen dem ersten Teil (2) und dem zweiten Teil (3) angeordnet ist, wobei das Beabstandungselement (6) derart ausgebildet ist, dass bei einer Schwenkbewegung des ersten Teils (2) relativ zum zweiten Teil (3) das Beabstandungselement (6) das Verbindungselement von einem Drehpol (7) der Schwenkbewegung beabstandet hält, wobei das Verbindungselement als Band (5) ausgebildet ist, wobei das Band (5) über das mindestens eine Beabstandungselement (6) verläuft, wobei eine biegsame Struktur (84) zum Bereitstellen der Schwenkbarkeit des ersten Teils (2) und des zweiten Teils (3) relativ zueinander das erste Teil (2) und das zweite Teil (3) verbindet, **dadurch gekennzeichnet, dass** die biegsame Struktur (84) in Form einer elastischen Platte (85) ausgebildet ist.

2. Vorrichtung (1) gemäß Anspruch 1, **dadurch gekennzeichnet, dass** die biegsame Struktur (84), das erste Teil (2) und/oder das zweite Teil (3) einstückig ausgebildet sind.

3. Vorrichtung (1) gemäß einem der vorstehenden Ansprüche, **dadurch gekennzeichnet, dass** eine Anbindung (20) des Verbindungselements, des Bandes (5), am ersten Teil (2) in Form einer Befestigung an dem ersten Teil (2) ausgebildet ist, oder dass die Anbindung (20) des Verbindungselements, des Bandes (5), am ersten Teil (2) in Form einer Rückführung vom ersten Teil (2) zurück zum zweiten Teil (3) ausgebildet ist, wobei die Rückführung derart ausgebildet ist, dass das Verbindungselement, das Band (5), am ersten Teil (2) umgelenkt und zum zweiten Teil (3) rückgeführt und am zweiten Teil (3) befestigt ist.

4. Vorrichtung (1) gemäß einem der vorstehenden Ansprüche, **dadurch gekennzeichnet, dass** ein Beabstandungselement (6) an dem ersten Teil (2) angeordnet ist und einteilig mit dem ersten Teil (2) ausgebildet ist, und/oder ein Beabstandungselement (6) an dem zweiten Teil (3) angeordnet ist und einteilig mit dem zweiten Teil (3) ausgebildet ist.

5. Vorrichtung (1) gemäß einem der vorstehenden Ansprüche, **dadurch gekennzeichnet, dass** das Beabstandungselement (6) separat zu dem ersten Teil (2) und dem zweiten Teil (3) bereitgestellt ist und zwischen dem ersten Teil (2) und dem zweiten Teil (3) angeordnet ist.

6. Vorrichtung (1) gemäß einem der vorstehenden Ansprüche, **dadurch gekennzeichnet, dass** ein Beabstandungselement (6) als Vorsprung ausgebildet ist, der sich von dem ersten Teil (2), zum zweiten Teil (3) erstreckt oder der sich von dem zweiten Teil (3), über das Körpergelenk zum ersten Teil (2) erstreckt.

7. Vorrichtung (1) gemäß einem der vorstehenden Ansprüche, **dadurch gekennzeichnet, dass** der erste Teil (2) und/oder der zweite Teil (3) mindestens eine Auflagefläche zum flächigen Kontaktieren des ersten beziehungsweise des zweiten Körperteils umfasst.

8. Vorrichtung (1) gemäß einem der vorstehenden Ansprüche, **dadurch gekennzeichnet, dass** die Vorrichtung (1) eine Handgelenkorthese ist, wobei der erste Teil (2) zur Befestigung an einer Hand eines Anwenders ausgebildet ist und der zweite Teil (3) zur Befestigung an einem Unterarm des Anwenders ausgebildet ist.

## Claims

1. Device (1) for damping body movement via a body joint, comprising a first part (2) for attachment to a first body part, a second part (3) pivotable relative to the first part (2) for attachment to a second body part located on the opposite side of the body joint in relation to the first body part, a damping element (4) attached to the second part (3), and a tensile-resistant connecting element, wherein the connecting element extends from the first part (2) to the second part (3), and wherein the tensile-resistant connecting element is attached to the first part (2) and is attached to the second part (3) via the damping element (4) to the second part (3), wherein at least one spacer element (6) is arranged between the first part (2) and the second part (3), wherein the spacer element (6) is designed such that, during a pivoting movement of the first part (2) relative to the second part (3), the spacer element (6) keeps the connecting element spaced apart from a pivot pole (7) of the pivoting movement, wherein the connecting element is designed as a strap (5), wherein the strap (5) runs over the at least one spacer element (6), wherein a flexible structure (84) for providing the pivotability of the first part (2) and the second part (3) relative to each other connects the first part (2) and the second part (3),
**characterized in that**
the flexible structure (84) is formed in the shape of an elastic plate (85).

2. Device (1) according to claim 1, **characterized in that** the flexible structure (84), the first part (2), and/or the second part (3) are formed in one piece.

3. Device (1) according to one of the preceding claims, **characterized in that** a connection (20) of the connecting element, the strap (5), to the first part (2) is formed in the form of a fastening to the first part (2), or that the connection (20) of the connecting element, the strap (5), on the first part (2) is designed in the form of a return from the first part (2) back to the second part (3), wherein the return is designed in such a way that the connecting element, the strap (5), is deflected on the first part (2) and returned to the second part (3) and fastened to the second part (3).

4. Device (1) according to one of the preceding claims, **characterized in that** a spacer element (6) is arranged on the first part (2) and is formed integrally with the first part (2), and/or a spacer element (6) is arranged on the second part (3) and is formed integrally with the second part (3).

5. Device (1) according to one of the preceding claims, **characterized in that** the spacer element (6) is provided separately from the first part (2) and the second part (3) and is arranged between the first part (2) and the second part (3).

6. Device (1) according to one of the preceding claims, **characterized in that** a spacer element (6) is designed as a projection extending from the first part (2) to the second part (3) or extending from the second part (3) via the body joint to the first part (2).

7. Device (1) according to one of the preceding claims, **characterized in that** the first part (2) and/or the second part (3) comprises at least one contact surface for flat contact with the first or the second body part.

8. Device (1) according to one of the preceding claims, **characterized in that** the device (1) is a wrist orthosis, wherein the first part (2) is designed for attachment to a hand of a user and the second part (3) is designed for attachment to a user's forearm.

## Revendications

1. Dispositif (1) d'amortissement d'un mouvement corporel par le biais d'une articulation corporelle, comprenant une première partie (2) pour la liaison avec une première partie corporelle, une seconde partie (3) pivotante par rapport à la première partie (2) pour la liaison avec une seconde partie corporelle placée par rapport à la première partie corporelle au-delà de l'articulation corporelle, un élément d'amortissement (4) fixé à la seconde partie (3) et un élément de liaison rigide à la traction, dans lequel l'élément de liaison s'étend de la première partie (2) à la seconde partie (3), et dans lequel l'élément de liaison rigide à la traction est lié à la première partie (2) et est fixé par le biais de l'élément d'amortissement (4) à la seconde partie (3), dans lequel au moins un élément d'espacement (6) est agencé entre la première partie (2) et la seconde partie (3), dans lequel l'élément d'espacement (6) est formé de telle manière que lors d'un mouvement de pivotement de la première partie (2) par rapport à la seconde partie (3), l'élément d'espacement (6) maintienne espacé l'élément de liaison d'un pôle de rotation (7) du mouvement de pivotement, dans lequel l'élément de liaison est formé comme bande (5), dans lequel la bande (5) s'étend sur l'au moins un élément d'espacement (6),
dans lequel une structure flexible (84) relie pour la fourniture de la pivotabilité de la première partie (2) et de la seconde partie (3) l'une par rapport à l'autre la première partie (2) et la seconde partie (3), **caractérisé en ce que** la structure flexible (84) est formée comme une plaque élastique (85).

2. Dispositif (1) selon la revendication 1, **caractérisé en ce que** la structure flexible (84), la première partie (2) et/ou la seconde partie (3) sont formées d'un seul tenant.

3. Dispositif (1) selon l'une quelconque des revendications précédentes, **caractérisé en ce qu'**une liaison (20) de l'élément de liaison, de la bande (5), avec la première partie (2) est formée comme une fixation à la première partie (2), ou **en ce que** la liaison (20) de l'élément de liaison, de la bande (5), avec la première partie (2) est formée comme un renvoi de la première partie (2) sur la seconde partie (3), dans lequel le renvoi est formé de telle manière que l'élément de liaison, la bande (5), soit déviée sur la première partie (2) et renvoyée par rapport à la seconde partie (3) et fixée à la seconde partie (3).

4. Dispositif (1) selon l'une quelconque des revendications précédentes, **caractérisé en ce qu'**un élément d'espacement (6) est agencé sur la première partie (2) et formé d'un seul tenant avec la première partie (2) et/ou un élément d'espacement (6) est agencé sur la seconde partie (3) et formé d'un seul tenant avec la seconde partie (3).

5. Dispositif (1) selon l'une quelconque des revendications précédentes, **caractérisé en ce que** l'élément d'espacement (6) est fourni séparément à la première partie (2) et la seconde partie (3) et agencé entre la première partie (2) et la seconde partie (3).

6. Dispositif (1) selon l'une quelconque des revendications précédentes, **caractérisé en ce qu'**un élément d'espacement (6) est formé comme saillie qui s'étend de la première partie (2), à la seconde partie (3) ou qui s'étend de la seconde partie (3) par le biais de l'articulation corporelle à la première partie (2).

7. Dispositif (1) selon l'une quelconque des revendications précédentes, **caractérisé en ce que** la première partie (2) et/ou la seconde partie (3) comprend au moins une surface d'appui pour la mise en contact à plat de la première ou de la seconde partie corporelle.

8. Dispositif (1) selon l'une quelconque des revendications précédentes, **caractérisé en ce que** le dispositif (1) est une orthèse de poignet, dans lequel la première partie (2) est formée pour la fixation à une main d'un utilisateur et la seconde partie (3) est formée pour la fixation à un avant-bras de l'utilisateur.
